# EUROPÄISCHE PATENTSCHRIFT

(11) **EP 0 784 617 B1**
(45) Veröffentlichungstag und Bekanntmachung des Hinweises auf die Patenterteilung: **10.07.2002**
(21) Anmeldenummer: 95935374.9
(22) Anmeldetag: 25.09.1995
(51) Int. Cl.: C07D 263/10, C07D 263/14, A01N 43/76, C07D 413/10, C07C 323/09, C07D 319/08, C07D 317/46, C07C 323/42, C07C 323/47, C07C 323/63

(54) **SUBSTITUIERTE BIPHENYLOXAZOLINE**
SUBSTITUTED DIPHENYL OXAZOLINES
OXAZOLINES DE BIPHENYLE SUBSTITUEES

(30) Priorität: 06.10.1994 DE 4435716; 23.06.1995 DE 19523388
(43) Veröffentlichungstag der Anmeldung: 23.07.1997
(73) Patentinhaber: BAYER AG, 51368 Leverkusen (DE)
(72) Erfinder: LANTZSCH, Reinhard, D-42115 Wuppertal (DE); MARHOLD, Albrecht, D-51373 Leverkusen (DE); ERDELEN, Christoph, D-42799 Leichlingen (DE)
(86) Internationale Anmeldenummer: EP9503787
(87) Internationale Veröffentlichungsnummer: WO9611190

(56) Entgegenhaltungen:
- EP-A- 0 345 775
- EP-A- 0 432 661
- WO-A-93/25079
- WO-A-95/04726
- DE-A- 1 643 382
- LU-A- 67 781

## Beschreibung

Die Erfindung betrifft neue substituierte Biphenyloxazoline, mehrere Verfahren und Zwischenprodukte zu ihrer Herstellung, und ihre Verwendung zur Bekämpfung von tierischen Schädlingen.

Es ist bekannt, daß bestimmte substituierte Biphenyloxazoline, wie 2-(2,6-Difluorphenyl)-4-(4'-trifluormethylthiobiphenyl-4)-2-oxazolin insektizid und akarizid wirksam sind (vgl. WO 95/04726). In WO 93/25079, EP-A-345 775 und EP-A-432 661 werden substituierte 2-Phenyloxazoline mit insektizider und akarizider Wirkung beschrieben.

Die Wirkungshöhe und/oder Wirkungsdauer dieser bekannten Verbindung ist jedoch, insbesondere gegen bestimmte Organismen oder bei niedrigen Anwendungskonzentrationen nicht in allen Anwendungsgebieten völlig zufriedenstellend.

Es wurden neue substituierte Biphenyloxazoline der Formel (I) 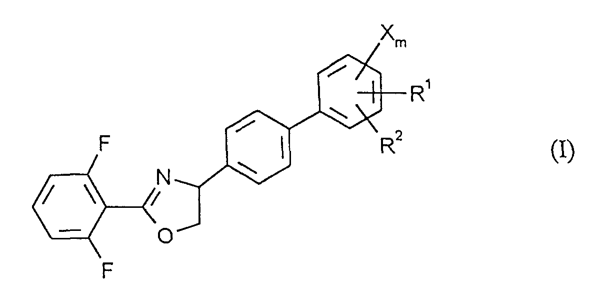 gefunden,
in welcher
- R¹: für C₁-C₆-Halogenalkylthio steht und
- R²: für Wasserstoff steht oder
- R¹ und R²: gemeinsam mit den Kohlenstoffatomen, an die sie gebunden sind einen halogensubstituierten 5- oder 6-gliedrigen heterocyclischen Ring bilden,
- X: für Wasserstoff, Halogen, C₁-C₆-Alkyl oder C₁-C₆-Alkoxy steht und
- m: für 0, 1 oder 2 steht,
ausgenommen die Verbindung der Formel 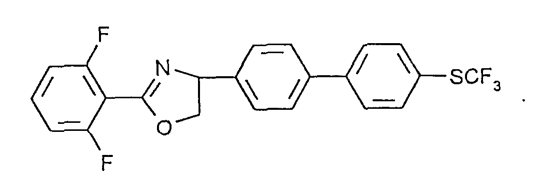 Aufgrund eines oder mehrerer Chiralitätszentren fallen die Verbindungen der Formel (I) im allgemeinen als Stereoisomerengemische an. Sie können sowohl in Form ihrer Diastereomerengemische als auch als reine Diastereomere oder Enantiomere verwendet werden.

Weiter wurde gefunden, daß man die neuen Verbindungen der Formel (I) erhält, wenn man

Verbindungen der Formel (II) 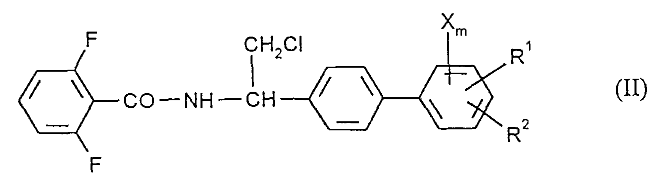 in welcher
- R¹, R², X und m: die oben angegebene Bedeutung haben, in Gegenwart einer Base, gegebenenfalls in Gegenwart eines Katalysators und gegebenenfalls in Gegenwart eines Verdünnungsmittels cyclisiert (Verfahren A).

Weiter wurde gefunden, daß die neuen substituierten Biphenyloxazoline der Formel (I) sehr gut zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- R¹: steht bevorzugt für C₁-C₄-Halogenalkylthio, und
- R²: für Wasserstoff oder

- R¹ und R²: bilden gemeinsam mit den einander direkt benachbarten Kohlenstoffatomen, an die sie gebunden sind, einen sauerstoffhaltigen 5- oder 6-gliedrigen Ring, der einfach oder mehrfach durch Fluor und/oder Chlor substituiert ist.
- X: steht bevorzugt für Fluor oder Chlor,
- m: steht bevorzugt für 0 oder 1.
- R¹: steht besonders bevorzugt für SCHF₂, SCF₂CHFCl, SCF₂CF₂H, SCF₂Cl, SCF₂Br, SCF₂CH₂F, SCF₂CF₃, SCF₂CHCl₂, SCH₂CF₂CHF₂, SCH₂CF₂CF₃ oder SCF₂CHFCF₃, und
- R²: für Wasserstoff oder,
- R¹ und R²: sind an direkt benachbarte Kohlenstoffatome gebunden und stehen gemeinsam für -OCF₂O-, -OCF₂CF₂O-, -OCF₂CFClO-, -OCF₂OCF₂ oder -OCF₂CF₂-.

- X: steht besonders bevorzugt für Fluor oder Chlor.
- m: steht besonders bevorzugt für 0 oder 1.

Ganz besonders bevorzugt sind diejenigen Verbindungen der Formel (I), in welchen R¹ in der 4-Position des Phenylrings gebunden ist.

Dabei ist die Verbindung der Formel 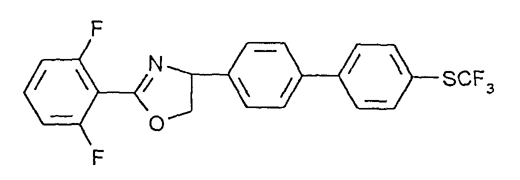 jeweils ausgenommen.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Verwendet man z.B. N-(1-(4-Tetrafluorethylthiobiphenyl-4)-2-chlor-ethyl-1)-2,6-difluorbenzamid als Ausgangsstoff, so kann der Verlauf des erfindungsgemäßen Verfahrens A durch das folgende Reaktionsschema wiedergegeben werden:

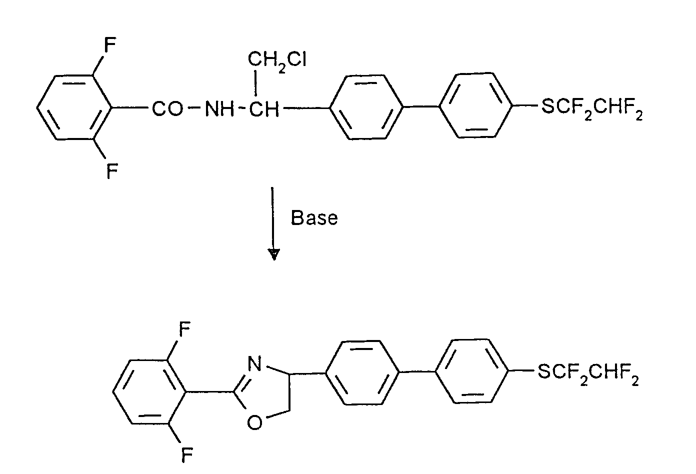

Das Verfahren A zur Herstellung der Verbindungen der Formel (I) ist dadurch gekennzeichnet, daß man die Verbindungen der Formel (II) in Gegenwart einer Base, gegebenenfalls in Gegenwart eines Katalysators und gegebenenfalls in Gegenwart eines Verdünnungsmittels cyclisiert.

Die Cyclisierung wird vorzugsweise in Gegenwart eines Verdünnungsmittels durchgeführt.

Als Verdünnungsmittel kommen alle inerten organischen Lösungsmittel in Frage. Sie können gegebenenfalls in Mischung mit Wasser verwendet werden. Bevorzugt verwendet werden Kohlenwasserstoffe wie Toluol, Xylol, Tetralin, Hexan, Cyclohexan, Halogenkohlenwasserstoffe wie Methylenchlorid, Chloroform, Chlorbenzol, o-Dichlorbenzol, Alkohole wie Methanol, Ethanol, Glykol, die isomeren Propanole, Butanole, Pentanole, Ether wie Diethylether, Diisopropylether, Dimethoxyethan, Tetrahydrofuran, Dioxan, Nitrile wie Acetonitril oder Butyronitril, Amide wie Dimethylformamid, Sulfoxide wie Dimethylsulfoxid, ferner Sulfolan. Besonders bevorzugt werden Alkohole verwendet.

Als Base kommen alle üblichen Säureakzeptoren in Frage.

Vorzugsweise verwendbar sind tertiäre Amine wie Triethylamin, Pyridin, DABCO, DBU, DBN, N,N-Dimethylanilin, ferner Erdalkalimetalloxide wie Magnesium- und Calciumoxid, außerdem Alkali- und Erdalkalimetallcarbonate wie Natriumcarbonat, Kaliumcarbonat und Calciumcarbonat, Alkalihydroxide wie Natrium- und Kaliumhydroxid, ferner Alkoholate wie Natriumethanolat oder Kalium-tert.-butylat.

Gegebenenfalls wird in Gegenwart eines Phasentransferkatalysators gearbeitet. Als Phasentransferkatalysator kommen beispielsweise Ammoniumverbindungen wie Tetraoctylammoniumbromid oder Benzyltriethylammoniumchlorid in Frage.

Die Temperatur kann in einem größeren Bereich variiert werden. Im allgemeinen arbeitet man bei Temperaturen zwischen -10°C und 150°C, bevorzugt zwischen 0°C und 100°C.

Die Umsetzung wird im allgemeinen unter Normaldruck durchgeführt.

Im allgemeinen wird eine äquimolare Menge an Base eingesetzt. Es ist aber gegebenenfalls auch möglich, mit einem Basenüberschuß zu arbeiten.

Es wird in üblicher Weise aufgearbeitet.

Die bei der Herstellung der Verbindungen der Formel (I) benötigten Ausgangsstoffe der Formel (II) sind neu; man erhält sie, wenn man Verbindungen der Formel (III) 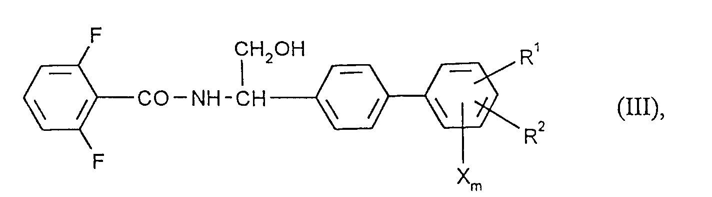 in welcher
R¹, R², X und m die oben angegebene Bedeutung haben, mit einem Chlorierungsmittel, gegebenenfalls in Gegenwart eines Verdünnungsmittels umsetzt (Verfahren B).

Verwendet man z.B. N-(1-(4'-Tetrafluorethylthiobiphenyl-4)-ethyl-2-ol)-2,6-difluorbenzamid und Thionylchlorid als Ausgangsstoffe, so kann der Verlauf nach Verfahren B durch das folgende Reaktionsschema wiedergegeben werden:

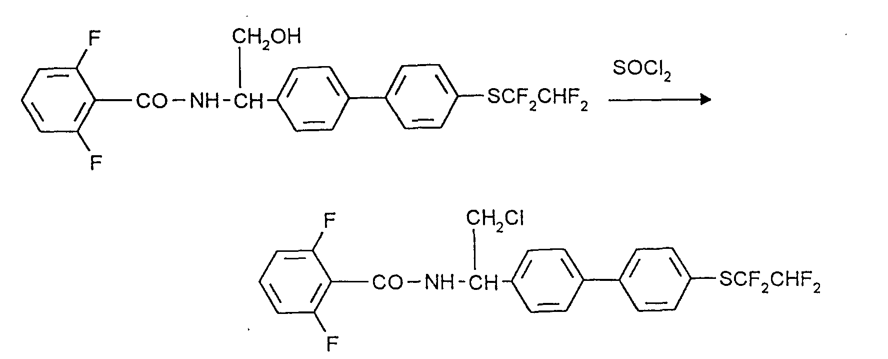

Das Verfahren B zur Herstellung von Verbindungen der Formel (II) ist dadurch gekennzeichnet, daß man Verbindungen der Formel (III) mit einem Chlorierungsmittel, gegebenenfalls in Gegenwart eines Verdünnungsmittels umsetzt.

Als Verdünnungsmittel kommen alle inerten organischen Lösungsmittel in Frage. Bevorzugt verwendet werden Kohlenwasserstoffe wie Toluol, Xylol, Hexan, Cyclohexan, Halogenkohlenwasserstoffe wie Chlorbenzol, Chloroform, Methylenchlorid und Ether wie Diethylether, Diisopropylether, Dimethoxyethan, Tetrahydrofuran, Dioxan.

Als Chlorierungsmittel kommen alle für diesen Zweck üblicherweise verwendbaren Reagenzien in Frage. Genannt seien beispielsweise Thionylchlorid, Phosgen und Phosphoroxychlorid, die im allgemeinen in mindestens äquimolarer Menge eingesetzt werden.

Die Temperatur kann in einem größeren Bereich variiert werden. Im allgemeinen arbeitet man bei Temperaturen zwischen 0°C und 120°C, bevorzugt zwischen 20°C und 100°C.

Die Umsetzung wird gegebenenfalls in Gegenwart einer Base, insbesondere eines tertiären Amins, beispielsweise Triethylamin oder Pyridin, durchgeführt.

Die Ausgangsstoffe der Formel (III) sind neu; man erhält sie beispielsweise, wenn man Verbindungen der Formel (IV) 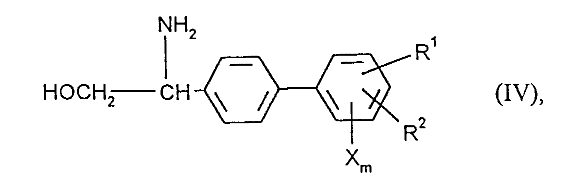 in welcher
- R¹, R², X und m: die oben angegebene Bedeutung haben,
mit 2,6-Difluorbenzoylchlorid gegebenenfalls in Gegenwart einer Base und gegebenenfalls in Gegenwart eines Verdünnungsmittels umsetzt (Verfahren C).

Verwendet man z.B. 2-Amino-2-(4'-Tetrafluorethylthiobiphenyl)-4)-ethan-1-ol als Ausgangsmaterial, so kann der Verlauf nach Verfahren C durch das folgende Reaktionsschema wiedergegeben werden:

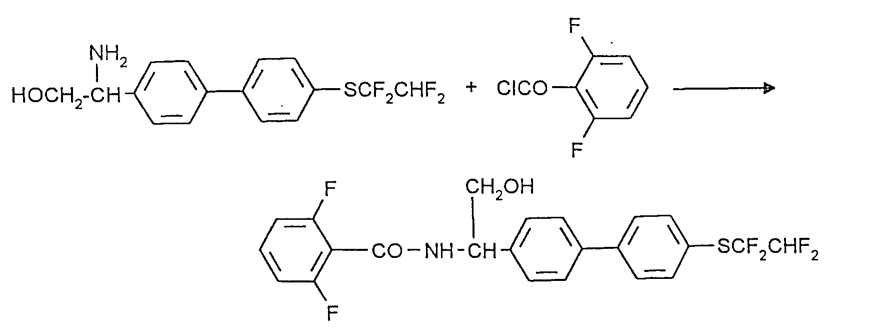

Das Verfahren C zur Herstellung von Verbindungen der Formel (III) ist dadurch gekennzeichnet, daß man Verbindungen der Formel (IV) gegebenenfalls in Gegenwart einer Base und gegebenenfalls in Gegenwart eines Verdünnungsmittels mit 2,6-Difluorbenzoylchlorid umsetzt.

Als Verdünnungsmittel können alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung alle üblichen Säureakzeptoren in Betracht. Vorzugsweise verwendbar sind tertiäre Amine, wie Triethylamin, Pyridin, Diazabiyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat und Alkali- oder Erdalkalihydroxide wie Natriumhydroxid oder Kaliumhydroxid.

Die Reaktionstemperaturen können innerhalb eines größeren Bereiches variiert werden. Im allgemeinen arbeitet man bei Temperaturen zwischen -20°C und +100°C, vorzugsweise zwischen 0°C und 30°C.

Die Umsetzung wird im allgemeinen unter Normaldruck durchgeführt.

Bei der Durchführung werden die Ausgangsstoffe der Formel (IV) und 2,6-Difluorbenzoylchlorid im allgemeinen in angenähert äquivalenten Mengen verwendet. Es ist jedoch auch möglich, das Carbonsäurehalogenid in einem größeren Überschuß (bis zu 5 mol) einzusetzen. Die Aufarbeitung erfolgt nach üblichen Methoden.

Die Ausgangsstoffe der Formel (IV) sind neu, man erhält sie, wenn man Verbindungen der Formel (V) 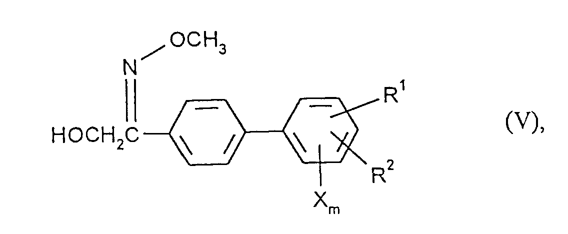 in welcher
- R¹, R², X und m: die oben angegebene Bedeutung haben,
mit einem Reduktionsmittel in Gegenwart einer Säure und gegebenenfalls in Gegenwart eines Verdünnungsmittels reduziert (Verfahren D).

Verwendet man z.B. 4-Hydroxyacetyl-oxim-O-methylether-4'-tetrafluorethylthiobiphenyl als Ausgangsstoff, so kann der Verlauf nach Verfahren D durch das folgende Reaktionsschema wiedergegeben werden:

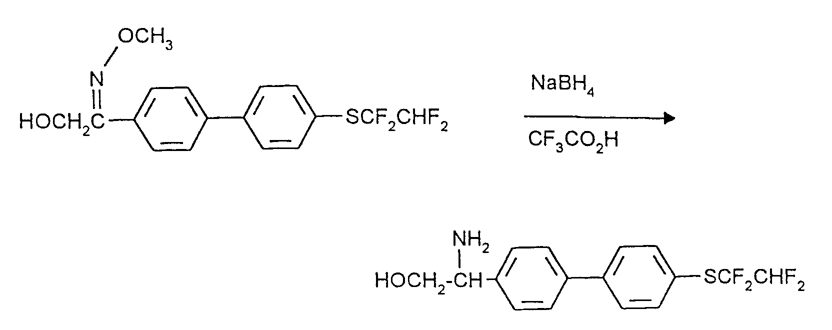

Das Verfahren D zur Herstellung von Verbindungen der Formel (IV) ist dadurch gekennzeichnet, daß man die Verbindung der Formel (V) mit einem Reduktionsmittel in Gegenwart einer Säure und gegebenenfalls in Gegenwart eines Verdünnungsmittels umsetzt.

Als Verdünnungsmittel kommen alle gegenüber den Reaktionsteilnehmern inerten Lösungsmittel in Frage. Vorzugsweise verwendet werden Ether, wie z.B. Diisopropylether, Methyl-tert-butylether, Tetrahydrofuran, 1,2-Dimethoxyethan, Dioxan.

Als Reduktionsmittel wird bevorzugt Natriumboranat in einer äquimolaren Menge oder im Überschuß verwendet.

Als Säure wird bevorzugt Trifluoressigsäure in einer äquimolaren Menge oder im Überschuß verwendet.

Die Temperatur kann in einem größeren Bereich variiert werden. Im allgemeinen arbeitet man zu Beginn der Umsetzung bei Temperaturen zwischen 0°C und 50°C und erhöht die Temperatur im Verlauf der Umsetzung gegebenenfalls auf bis zu 120°C.

Die Umsetzung wird im allgemeinen unter Normaldruck durchgeführt.

Die Aufarbeitung erfolgt mit Hilfe üblicher Methoden.

Das Reaktionsprodukt der Formel (IV) wird vorzugsweise in Form von Salzen, beispielsweise der Hydrochloride, isoliert.

Die Zwischenprodukte der Formel (V) sind neu, man erhält sie, wenn man Verbindungen der Formel (VI) 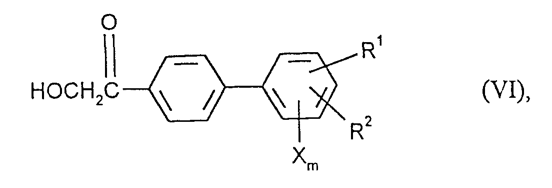 in welcher
- R¹, R², X und m: die oben angegebene Bedeutung haben,
mit der Verbindung der Formel (VII)

H₂N-OCH₃ (VII)

gegebenenfalls in Gegenwart eines Verdünnungsmittels umsetzt (Verfahren E).

Verwendet man z.B. 4-Hydroxyacetyl-4'-tetrafluorethylthiobiphenyl als Ausgangsstoff, so kann der Verlauf nach Verfahren E durch das folgende Reaktionsschema wiedergegeben werden:

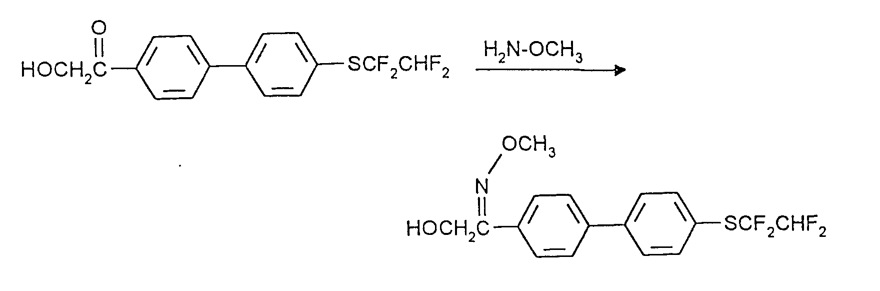

Das Verfahren E zur Herstellung von Verbindungen der Formel (V) ist dadurch gekennzeichnet, daß man Verbindungen der Formel (VI) mit der Verbindung der Formel (VII) gegebenenfalls in Gegenwart eines Verdünnungsmittels umsetzt.

Als Verdünnungsmittel kommen alle üblichen Lösungsmittel in Frage. Vorzugsweise verwendet werden beispielsweise Alkohole wie Methanol, Ethanol, die isomeren Propanole, Butanole, Pentanole, oder Ether wie Diisopropylether, Tetrahydrofuran, Dioxan, die gegebenenfalls im Gemisch mit Wasser eingesetzt werden können.

O-Methylhydroxylamin der Formel (VII) kann als freie Base oder auch als Salz einer Säure eingesetzt werden. Im letzteren Fall arbeitet man in Gegenwart einer Base, vorzugsweise Natriumacetat. Die Verbindung der Formel (VII) wird im allgemeinen in äquimolaren Mengen eingesetzt.

Die Temperatur kann in einem größeren Bereich variiert werden. Im allgemeinen arbeitet man bei Temperaturen zwischen -20°C und 100°C, bevorzugt zwischen 0°C und 60°C.

Die Umsetzung wird im allgemeinen unter Normaldruck durchgeführt.

Die Aufarbeitung erfolgt in üblicher Weise z.B. durch Filtration oder Extraktion.

Die Zwischenprodukte der Formel (VI) sind neu, man erhält sie, wenn man Verbindungen der Formel (VIII) 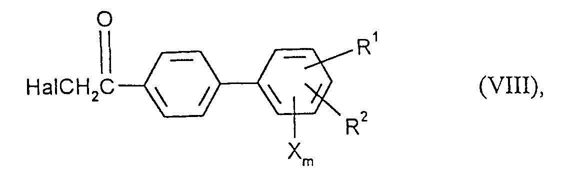 in welcher
- R¹, R², X und m: die oben angegebene Bedeutung haben und
- Hal: für Chlor oder Brom steht,
gegebenenfalls in Gegenwart eines Katalysators mit einem Salz der Ameisensäure umsetzt (Verfahren F).

Verwendet man 4-Chloracetyl-4'-trifluormethylthiobiphenyl als Ausgangsstoff, so kann der Verlauf nach Verfahren F durch das folgende Formelschema wiedergegeben werden:

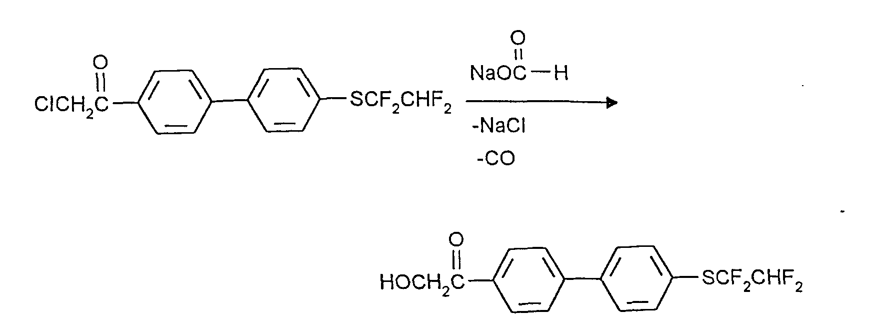

Das Verfahren F zur Herstellung von Verbindungen der Formel (VI) ist dadurch gekennzeichnet, daß man Verbindungen der Formel (VIII) mit einem Salz der Ameisensäure gegebenenfalls in Gegenwart eines Katalysators umsetzt.

Als Verdünnungsmittel kommen alle üblichen, unter den Reaktionsbedingungen inerten Lösungsmittel in Frage. Vorzugsweise verwendbar sind Kohlenwasserstoffe wie Toluol, Xylol, Mesitylen, Cyclohexan, Methylcyclohexan, Chlorkohlenwasserstoffe wie Chlorbenzol, o-Dichlorbenzol, Alkohole wie Methanol, Ethanol, die isomeren Propanole, die isomeren Butanole und Pentanole, Ether wie Diisopropylether, Tetrahydrofuran, Dioxan, Nitrile wie Acetonitril und Butyronitril, Amide wie Dimethylformamid und auch stark polare Solventien wie Dimethylsulfoxid und Sulfolan.

Die genannten Verdünnungsmittel können gegebenenfalls auch im Gemisch mit Wasser verwendet werden, gegebenenfalls in Gegenwart eines Phasentransferkatalysators, wie quartären Ammoniumsalzen, wie Tetraoctylammoniumbromid oder Benzyltriethylammoniumchlorid.

Vorzugsweise verwendbare Salze der Ameisensäure sind Natriumformiat und Kaliumformiat.

Die Temperatur kann in einem größeren Bereich variiert werden. Im allgemeinen arbeitet man bei Temperaturen zwischen 50°C und 200°C, bevorzugt zwischen 80°C und 160°C.

Im allgemeinen geht man so vor, daß man die Verbindung der Formel (VIII) mit 1 bis 20 mol, bevorzugt 1 bis 5 mol Formiat in einem Verdünnungsmittel erhitzt, gegebenenfalls Wasser zugibt, die Phasen trennt und das Verdünnungsmittel abdestilliert.

Die Zwischenprodukte der Formel (VIII) sind neu; man erhält sie, wenn man Verbindungen der Formel (IX) 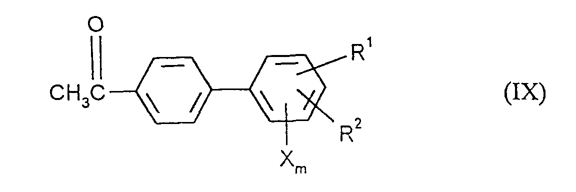 in welcher
- R¹, R², X und m: die oben angegebene Bedeutung haben
gegebenenfalls in Gegenwart eines Verdünnungsmittels chloriert oder bromiert (Verfahren G).

Verwendet man z.B. 2,2,3,3-Tetrafluor-5-(4-acetylphenyl)-dihydrobenzofuran- als Ausgangsstoff, so kann der Verlauf nach Verfahren G durch das folgende Formelschema wiedergegeben werden:

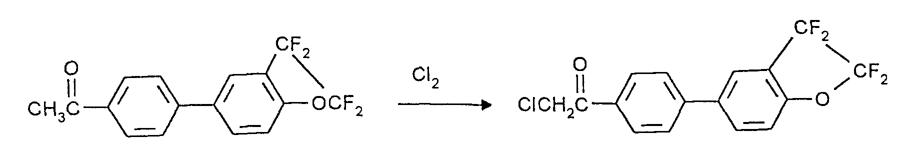

Das Verfahren G zur Herstellung von Verbindungen der Formel (VIII) ist dadurch gekennzeichnet, daß man Verbindungen der Formel (IX) gegebenenfalls in Gegenwart eines Verdünnungsmittels chloriert oder bromiert.

Als Verdünnungsmittels kommen alle gegenüber Chlor und Brom inerten Lösungsmittel in Frage. Bevorzugt verwendet werden beispielsweise Chlorkohlenwasserstoffe wie Methylenchlorid, Chloroform oder Tetrachlorkohlenstoff oder Alkohole wie Methanol oder Ethanol.

Die Temperatur kann innerhalb eines größeren Bereichs variiert werden. Im allgemeinen arbeitet man bei einer Temperatur zwischen -30°C und 50°C, bevorzugt zwischen -10°C und 25°C.

Die Umsetzung wird im allgemeinen unter Normaldruck durchgeführt.

Im allgemeinen geht man so vor, daß die Verbindung der Formel (IX) in einem geeigneten Verdünnungsmittel vorgelegt wird und dann bei der gewünschten Temperatur eine etwa äquimolare Menge Chlor oder Brom zudosiert wird. Man kann auch einen geringen Über- oder Unterschuß Halogen einsetzen.

Die Zwischenprodukte der Formel (IX) sind neu; man erhält sie, wenn man Verbindungen der Formel (X) 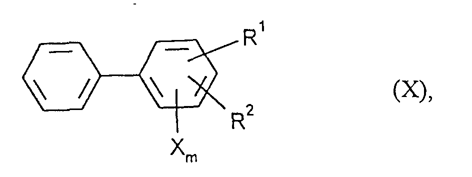 in welcher
- R¹, R², X und m: die oben angegebene Bedeutung haben
gegebenenfalls in Gegenwart einer Säure oder Lewis-Säure und in Gegenwart eines Verdünnungsmittels mit Acetylchlorid umsetzt (Verfahren H).

Verwendet man z.B. 2,2-Difluor-5-phenyl-benzodioxol als Ausgangsstoff, so kann der Verlauf nach Verfahren H durch das folgende Reaktionsschema dargestellt werden:

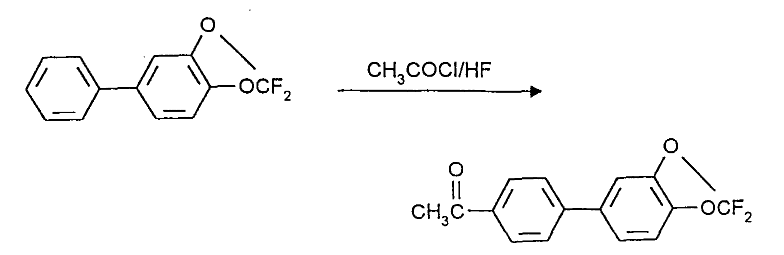

Das Verfahren H zur Herstellung von Verbindungen der Formel (IX) ist dadurch gekennzeichnet, daß man Verbindungen der Formel (X) in Gegenwart einer Säure oder Lewis-Säure und in Gegenwart eines Verdünnungsmittels mit Acetylchlorid umsetzt.

Als Verdünnungsmittel kommen alle üblichen, für Friedel-Crafts-Reaktionen geeigneten Lösungsmittel in Frage. Bevorzugt verwendet werden chlorierte Kohlenwasserstoffe wie z.B. Methylenchlorid oder Dichlorethan oder es wird in überschüssiger wasserfreier Flußsäure gearbeitet.

Als Säure oder Lewis-Säure kommen alle für Friedel-Crafts-Reaktionen geeigneten in Frage. Bevorzugt verwendet werden wasserfreie Flußsäure, Aluminiumchlorid, Tetrafluoroborsäure oder BF₃-Etherat.

Die Temperatur kann innerhalb eines größeren Bereichs variiert werden. Im allgemeinen arbeitet man bei Temperaturen zwischen -30°C und 80°C, bevorzugt zwischen -15°C und 50°C.

Die Umsetzung wird im allgemeinen unter Normaldruck oder unter erhöhtem Druck, der sich beim Arbeiten mit HF einstellt, durchgeführt.

Acetylchlorid und die Verbindungen der Formel (X) werden im allgemeinen in etwa äquimolaren Mengen eingesetzt.

Nach beendeter Umsetzung wird mit Hilfe üblicher Methoden aufgearbeitet.

Die Zwischenprodukte der Formel (X) sind neu; man erhält sie, wenn man Verbindungen der Formel (XI) 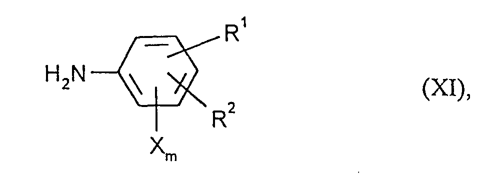 in welcher
- R¹, R², X und m: die oben angegebene Bedeutung haben,
diazotiert und das entstandene Diazoniumsalz in Gegenwart einer Säure und Eisenpulver oder in Gegenwart einer Base und jeweils in Gegenwart eines Verdünnungsmittels mit Benzol umsetzt.

Die Aniline der Formel (XI) sind bekannt und/oder lassen sich nach bekannten Methoden in einfacher Weise herstellen. Man erhält die Verbindungen der Formel (XI) beispielsweise, indem man die entsprechenden Nitroaromaten reduziert oder die entsprechenden Carbonsäureamide beispielsweise einem Hofmann-Abbau oder dergleichen unterwirft (Verfahren I).

Verwendet man z.B. Tetrafluorethylmercaptoanilin als Ausgangsstoff, so kann der Verlauf nach Verfahren I durch das folgende Reaktionsschema wiedergegeben werden:

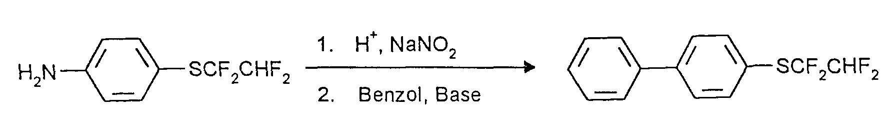

Das Verfahren I zur Herstellung von Verbindungen der Formel (X) ist dadurch gekennzeichnet, daß man Verbindungen der Formel (XI) diazotiert und in Gegenwart von Säure und Eisenpulver oder in Gegenwart einer Base und gegebenenfalls in Gegenwart eines Verdünnungsmittels mit Benzol umsetzt.

Als Verdünnungsmittel kommen alle inerten Lösungsmittel in Frage. Es kann aber auch ein größerer Überschuß des Reaktionspartners Benzol, vorzugsweise bis zu 30 mol, besonders bevorzugt bis zu 5 mol, bezogen auf die Verbindung der Formel (XI), als Verdünnungsmittel verwendet werden.

Wird die Umsetzung in Gegenwart von Säure und Eisenpulver durchgeführt, kommen als Säure organische Säuren, wie z.B. Trichloressigsäure, in Frage.

Wird die Umsetzung in Gegenwart einer Base durchgeführt, kommen als Base z.B. Salze organischer Säuren, wie Alkaliacetate, insbesondere Natriumacetat oder Kaliumacetat, in Frage.

Im allgemeinen werden zwei Äquivalente an Base eingesetzt.

Die Temperatur kann innerhalb eines größeren Bereichs variiert werden. Im allgemeinen arbeitet man bei einer Temperatur zwischen -40°C und 140°C, bevorzugt zwischen -20°C und 80°C.

Die Umsetzung wird im allgemeinen unter Normaldruck durchgeführt.

Das Diazoniumsalz wird im allgemeinen in Gegenwart einer Säure wie Salzsäure oder Schwefelsäure aus der Verbindung der Formel (XI) in üblicher Weise durch Umsetzung mit einem Alkalinitrit wie Natriumnitrit oder einem Alkylnitrit wie Pentylnitrit oder Methylnitrit oder durch Umsetzung mit Nitrosylchlorid hergestellt.

Das das Produkt der Formel (X) enthaltende Reaktionsgemisch wird mit Hilfe üblicher Methoden aufgearbeitet.

Ein weiteres Verfahren zur Herstellung der Zwischenprodukte der Formel (VIII) besteht darin, daß man Verbindungen der Formel (X) 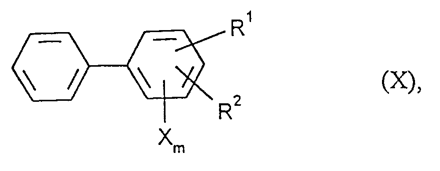 in welcher
- R¹, R², X und m: die oben angegebene Bedeutung haben
mit Halogenacetylchloriden der Formel (XII)

HalCH₂COCl (XII),

in welcher
- Hal: für Chlor oder Brom steht,
in Gegenwart einer Säure oder Lewis-Säure und in Gegenwart eines Verdünnungsmittels umsetzt (Verfahren K).

Verwendet man z.B. 2,2,4,4,-Tetrafluor-6-phenyl-1,3-benzodioxen und Chloracetylchlorid als Ausgangsstoffe, so kann der Verlauf nach Verfahren K durch das folgende Formelschema wiedergegeben werden:

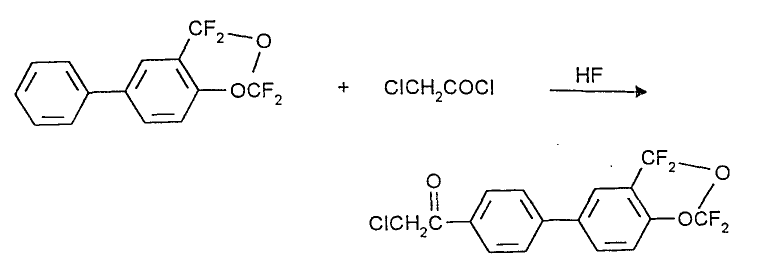

Das Verfahren K zur Herstellung von Verbindungen der Formel (VIII) ist dadurch gekennzeichnet, daß man Verbindungen der Formel (X) mit Halogenacetylchloriden der Formel (XII) in Gegenwart einer Säure oder Lewis-Säure und in Gegenwart eines Verdünnungsmittels umsetzt.

Als Verdünnungsmittel kommen alle üblichen, für Friedel-Crafts-Reaktionen geeigneten Lösungsmittel in Frage. Bevorzugt verwendet werden chlorierte Kohlenwasserstoffe wie z.B. Methylenchlorid oder Dichlorethan oder es wird in überschüssiger wasserfreier Flußsäure gearbeitet.

Als Säuren oder Lewis-Säuren kommen alle für Friedel-Crafts-Reaktionen geeigneten in Frage. Bevorzugt verwendet werden wasserfreie Flußsäure, Aluminiumchlorid oder Tetrafluoroborsäure.

Die Temperatur kann innerhalb eines größeren Bereichs variiert werden. Im allgemeinen arbeitet man zwischen -30°C und 80°C, bevorzugt zwischen -15°C und 50°C.

Die Umsetzung wird im allgemeinen unter Normaldruck oder unter erhöhtem Druck, der sich beim Arbeiten mit HF einstellt, durchgeführt.

Das Halogenacetylchlorid der Formel (XII) und die Verbindung der Formel (X) werden im allgemeinen in etwa äquimolaren Mengen eingesetzt.

Nach beendeter Umsetzung wird mit Hilfe üblicher Methoden aufgearbeitet.

Man erhält die Ausgangsstoffe der Formel (III) auch, wenn man Verbindungen der Formel (X) 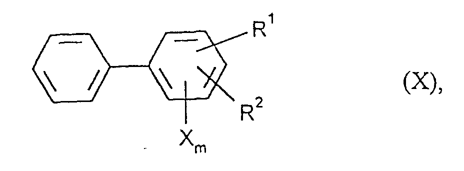 in welcher
- R¹, R², X und m: die oben angegebene Bedeutung haben
mit Verbindungen der Formel (XIII) 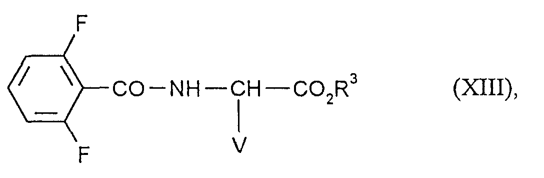 in welcher
- V: für Chlor, Hydroxy oder C₁-C₄-Alkoxy steht und
- R³: für Wasserstoff oder Alkyl (bevorzugt C₁-C₆-Alkyl) steht,
in Gegenwart eines sauren Katalysators und gegebenenfalls in Gegenwart eines Verdünnungsmittels umsetzt und die so erhaltenen Verbindungen der Formel (XIV) 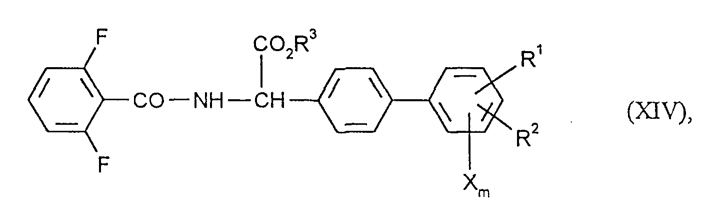 in welcher
- R¹, R², R³, X und m: die oben angegebene Bedeutung haben,
in Gegenwart eines Reduktionsmittels und in Gegenwart eines Verdünnungsmittels reduziert (Verfahren L).

Verwendet man z.B. N-(Carboxymethylchlormethyl)-2,6-difluorbenzamid und 2,2-Difluor-5-phenyl-benzodioxol als Ausgangsstoffe, so kann der Verlauf nach Verfahren L durch das folgende Reaktionsschema wiedergegeben werden:

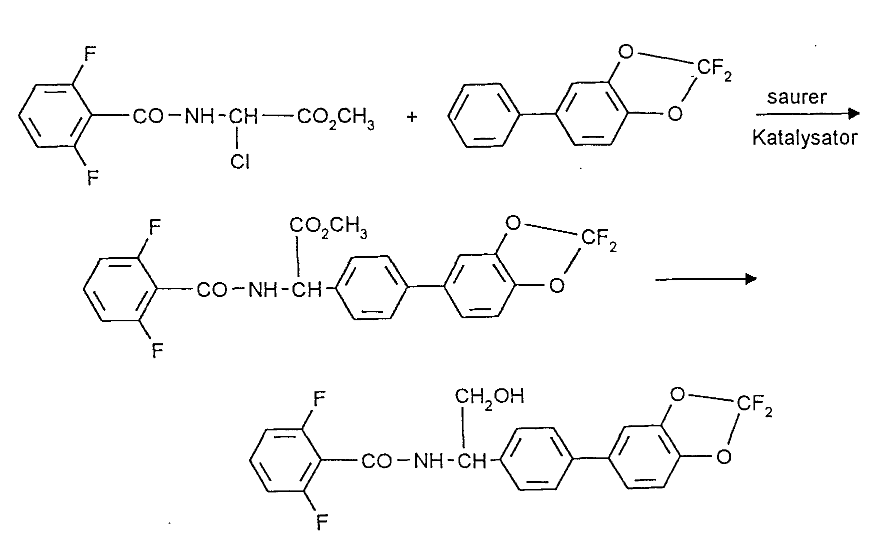

Das Verfahren L zur Herstellung der Verbindung der Formel (III) ist dadurch gekennzeichnet, daß man zunächst (1. Stufe) die Verbindung der Formel (X) mit einer Verbindung der Formel (VIII) in Gegenwart eines sauren Katalysators und gegebenenfalls in Gegenwart eines Verdünnungsmittels umsetzt und anschließend (2. Stufe) die so erhaltene Verbindung der Formel (XIV) mit einem Reduktionsmittel in Gegenwart eines Verdünnungsmittels umsetzt.

Als Verdünnungsmittel kommen in der ersten Stufe alle gegenüber den Reaktionsteilnehmern inerten Lösungsmittel in Frage.

Vorzugsweise verwendet werden Kohlenwasserstoffe wie Pentan, Hexan, Tetralin, Halogenkohlenwasserstoffe wie Methylenchlorid, Chloroform, Ether wie Diisopropylether, Methyl-tert-butylether, Tetrahydrofuran, Dioxan, Dimethoxyethan oder tert-Amylmethylether.

Als saurer Katalysator kommen prinzipiell alle anorganischen oder organischen Säuren oder Lewis-Säuren in Frage. Bevorzugt verwendet werden beispielsweise Schwefelsäure, Methansulfonsäure, Benzolsulfonsäure, wasserfreie Flußsäure, Tetrafluoroborsäure, Aluminiumchlorid, Titantetrachlorid, Phosphoroxychlorid, Bortrifluorid-Etherat. Bevorzugt kann auch ein Überschuß an Säure gegebenenfalls als Verdünnungsmittel dienen.

Die Temperatur kann innerhalb eines größeren Bereichs variiert werden. Im allgemeinen arbeitet man bei Temperaturen zwischen -20°C und 150°C, bevorzugt zwischen 0°C und 50°C.

Die Umsetzung wird im allgemeinen unter Normaldruck oder unter erhöhtem Druck durchgeführt.

Die Verbindung der Formel (X) und die Verbindung der Formel (XIII) werden im allgemeinen in äquimolaren Mengen eingesetzt, es ist jedoch auch möglich, einen Überschuß der einen oder anderen Verbindung zu verwenden.

Als Verdünnungsmittel kommen in der zweiten Stufe insbesondere Alkohole und Ether in Frage. Genannt seien beispielsweise Methanol, Ethanol, die isomeren Propanole, Butanole oder Pentanole, ferner Diethylether, Diisopropylether, Methyltert.-butylether, Tetrahydrofuran, Dioxan, Dimethoxyethan.

Als Reduktionsmittel wird bevorzugt Natriumborhydrid in einer Menge von 1 bis 5 mol, bezogen auf 1 mol der Verbindung der Formel (XIV).

Wenn die Verbindung der Formel (XIV) als Säure vorliegt (R³ = H), ist diese vor der Umsetzung mit Natriumborhydrid in einen Alkylester zu überführen.

Die Temperatur kann innerhalb eines größeren Bereichs variiert werden. Im allgemeinen arbeitet man bei Temperaturen zwischen 20°C und 150°C, bevorzugt zwischen 50°C und 100°C.

Die Umsetzung wird im allgemeinen unter Normaldruck durchgeführt.

Es wird mit Hilfe üblicher Methoden aufgearbeitet.

Ein weiteres Verfahren zur Herstellung der Verbindungen der Formel (II) besteht darin, daß man Verbindungen der Formel (X) 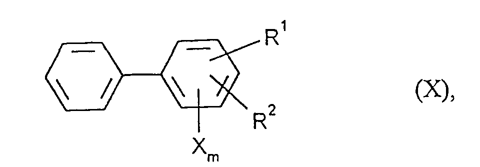 in welcher
- R¹, R², X und m: die oben angegebene Bedeutung haben,
mit Verbindungen der Formel (XV) 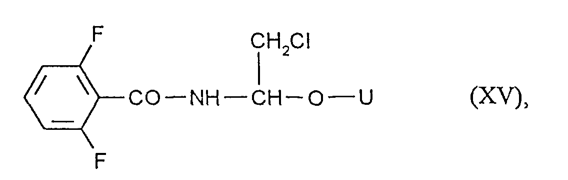 in welcher U für Alkyl (bevorzugt C₁-C₄-Alkyl) steht,
in Gegenwart eines Katalysators und gegebenenfalls in Gegenwart eines Verdünnungsmittels umsetzt (Verfahren M).

Verwendet man z.B. N-(1-Methoxy-2-chlorethyl)-2,6-difluorbenzamid und 4-Tetrafluorethylthiobiphenyl als Ausgangsstoffe, so kann der Verlauf nach Verfahren M durch das folgende Reaktionsschema wiedergegeben werden:

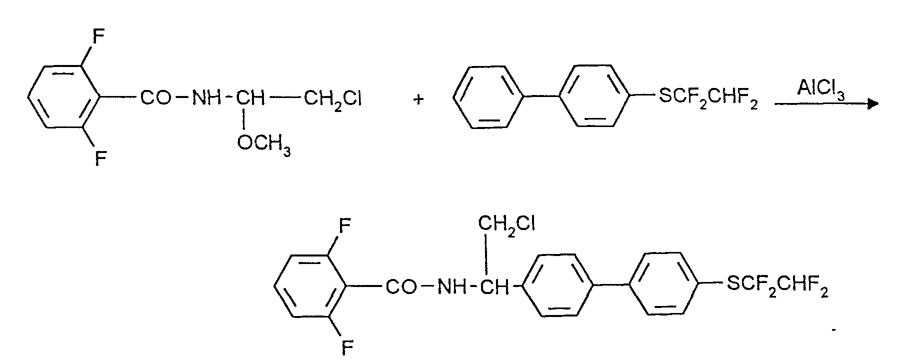

Das Verfahren M zur Herstellung von Verbindungen der Formel (II) ist dadurch gekennzeichnet, daß man Verbindungen der Formel (X) mit Verbindungen der Formel (XV) in Gegenwart eines sauren Katalysators und gegebenenfalls in Gegenwart eines Verdünnungsmittels umsetzt.

Als Verdünnungsmittel kommen alle gegenüber den Reaktionsteilnehmern inerten Lösungsmittel in Frage.

Vorzugsweise verwendet werden Kohlenwasserstoffe, wie Pentan, Hexan, Tetralin, Halogenkohlenwasserstoffe wie Methylenchlorid, Chloroform, Ether wie Diisopropylether, Methyl-tert.-butylether, Tetrahydrofuran, Dioxan, Dimethoxyethan, Methyl-tert.-amyl-ether.

Als saurer Katalysator kommen anorganische oder organische Säuren oder Lewis-Säuren in Frage. Bevorzugt verwendet werden beispielsweise Schwefelsäure, Methansulfonsäure, Benzolsulfonsäure, Tetrafluoroborsäure, Aluminiumchlorid, Titantetrachlorid, Phosphoroxychlorid, Bortrifluor-Etherat. Bevorzugt kann auch ein Überschuß an Säure gegebenenfalls als Verdünnungsmittel dienen.

Die Temperatur kann innerhalb eines größeren Bereichs variiert werden. Im allgemeinen arbeitet man bei Temperaturen zwischen -20°C und 150°C, bevorzugt zwischen 0°C und 50°C.

Die Umsetzung wird im allgemeinen unter Normaldruck oder unter erhöhtem Druck durchgeführt.

Die Verbindung der Formel (X) und die Verbindung der Formel (XV) werden im allgemeinen in äuqimolaren Mengen eingesetzt; es ist jedoch auch möglich, einen Überschuß der einen oder anderen Verbindung zu verwenden.

Die als Ausgangsstoffe benötigten Halogenacetylchloride der Formel (XII) sind gängige, allgemein bekannte Chemikalien der Organischen Chemie.

Die benötigte Verbindung (VII) ist eine allgemein bekannte Chemikalie der Organischen Chemie.

Die als Ausgangsstoffe benötigten Verbindungen der Formel (XIII) sind bekannt und/oder lassen sich nach bekannten Methoden in einfacher Weise herstellen (vgl. z.B. WO 93/24 470).

Die als Ausgangsstoffe benötigten Verbindungen der Formel (XV) sind bekannt und/oder lassen sich nach bekannten Methoden in einfacher Weise herstellen (vgl. z.B. EP-A-0 594 179).

Die Zwischenprodukte der Formeln (X), (IX), (VIII), (VI), (V), (IV), (III), (XIV) und (II) sind neu und ebenfalls Gegenstand der Erfindung. Sie weisen z.T. selbst insektizide und akarizide Eigenschaften auf, so beispielsweise die Verbindungen der Formel (III) und (II).

Die Umsetzung von bestimmten N-Alkoxymethylbenzamidderivaten mit Benzolderivaten in Gegenwart von beispielsweise konzentrierter Schwefelsäure oder Phosphoroxychlorid oder wasserfreiem Aluminiumchlorid zu den entsprechenden substituierten Phenylmethylbenzamidderivaten ist bekannt (siehe EP-A-0 594 179).

Die mit diesem bekannten Verfahren erzielbaren Ausbeuten sind jedoch nicht immer befriedigend.

Die Wirkstoffe der Formel (I) eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Raupen der Kohlschabe (Plutella maculipennis) oder gegen die Raupen des Eulenfalters (Spodoptera frugiperda) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox; Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivemectin, Lamda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301 / 5302, Zetamethrin.
**Herbizide:**
   beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die Herstellung und die Verwendung der erfindungsgemäßen Wirkstoffe geht aus den nachfolgenden Beispielen hervor.

### Herstellungsbeispiele

### Beispiel (I-1)

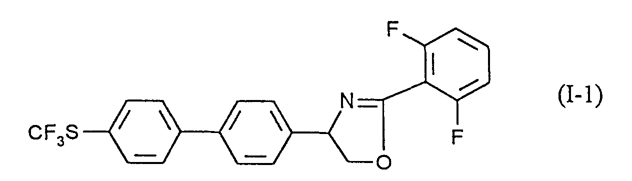

Man suspendiert 4,4 g (0,01 Mol) 2,6-Difluor-N[-2-chlorethyl-1-phenyl-4-(4'-trifluormethylthiophenyl)]-benzoesäureamid aus Beispiel (XIV-2) in 50 ml Methanol.

Dann gibt man 5,6 g (0,042 Mol) 30 %ige Natronlauge zu und erhitzt 20 Minuten auf 70°C. Nach Abkühlen engt man ein, nimmt in Methylenchlorid auf und wäscht dreimal mit Wasser. Nach Trocknen und Einengen bleiben 3,9 g gelbe Kristalle zurück, die über Kieselgel (Petrolether/Essigester 1:1) gereinigt werden. Man erhält 3,7 g farblose Kristalle vom Fp. 114°C.
Ausbeute: 91,8 % d.Th.

### Beispiel (I-2)

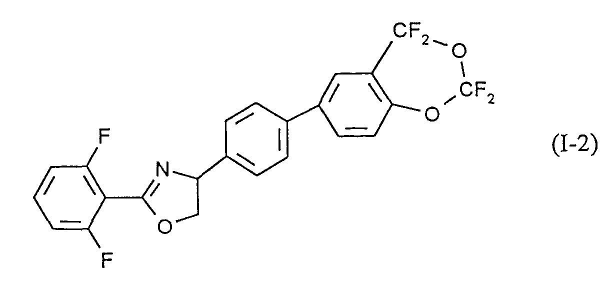

5,19 g (0,01 Mol) der Verbindung gemäß Beispiel (XIV-1) werden in 50 ml trockenem Methanol suspendiert. Man tropft ohne zu Kühlen 5,33 g (0,04 Mol) 30 %ige Natronlauge zu, wobei eine leicht exotherme Reaktion erfolgt. Man erhitzt 30 Min zum Sieden und kühlt ab. Nach Einengen löst man den Rückstand in Essigester, wäscht dreimal mit Wasser, trocknet und engt ein. Man erhält 3,7 g (79,5 % d. Th.) gelbe Kristalle vom Fp. 95-98°C.

### Beispiel (I-3)

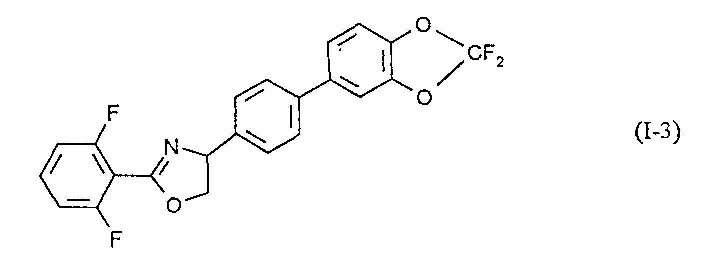

Das Produkt wird analog Beispiel (I-2) erhalten.

### Beispiel (I-4)

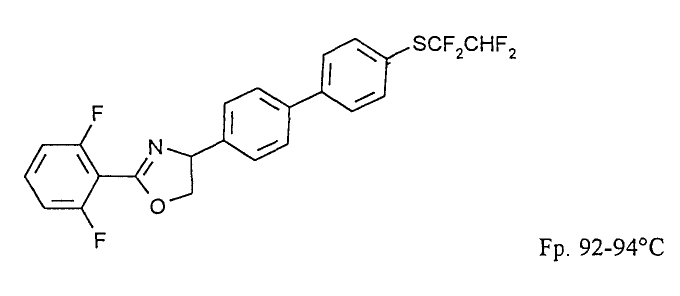

### Beispiel (I-5)

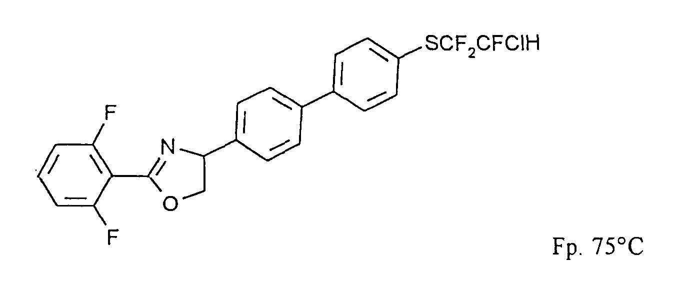

### Beispiel (I-6)

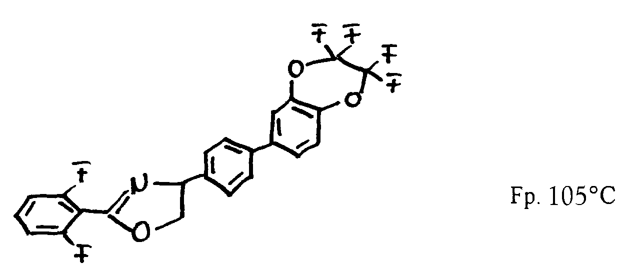

### Herstellung von Ausgangsverbindungen

### Beispiel (X-1)

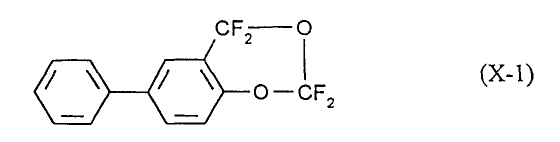

Zu 600 ml Benzol, 30 g Fe-Pulver und 336 g 2,2,4,4-Tetrafluor-6-amino-benzo-1,3-dioxen der Formel 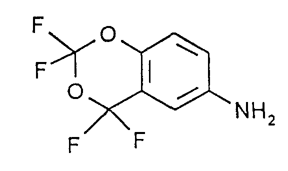 wird innerhalb von 5 Stunden bei 30-38°C eine Lösung von 750 g Trichloressigsäure in 1 200 ml Benzol zugetropft. Gleichzeitg werden 162 g Natriumnitrit (in Portionen von je 8 g alle 15 min) zugegeben. Nach beendeter Zugabe wird ca. 20 Stunden bei Raumtemperatur gerührt. Anschließend wird bis zum Ende der Gasentwicklung unter Rückfluß erhitzt (ca. 4 Stunden). Nach dem Abkühlen gibt man zunächst 1,8 1 5 %ige Salzsäure zu und destilliert dann überschüssiges Benzol ab, bis eine Innentemperatur von 90°C erreicht ist. Anschließend wird eine Wasserdampfdestillation durchgeführt. Die organische Phase wird abgetrennt, mit Wasser gewaschen, getrocknet und destilliert.
Man erhält 108 g der oben gezeigten Verbindung (X-1) vom Kp. (Siedepunkt) 135-141°C/15 mbar.

Analog und gemäß den allgemeinen Angaben zur Herstellung werden erhalten:

### Beispiel (X-2)

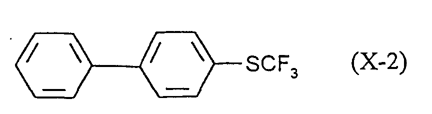

Fp.: 47-48°C
Kp. = 140 - 144°C/20 mbar

### Beispiel (X-3)

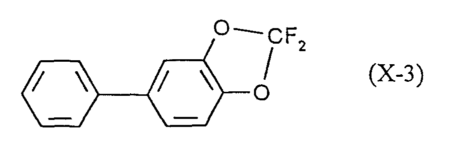

Fp.: 75°C
Kp.: 108-115°C (0,2 mbar)

### Beispiel (XIV-1)

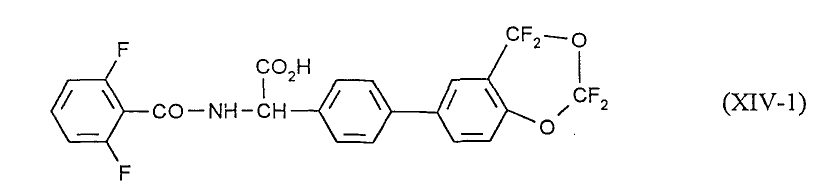

11,6 g (0,05 Mol) N-(2,6-Difluorbenzoyl)-2-hydroxy-glycin der Formel 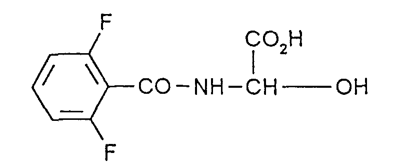 und 90 ml Methansulfonsäure werden bei 15°C vermischt. Man fügt 14,2 g (0,05 Mol) 2,2,4,4,-Tetrafluor-6-phenyl-1,3-benzodioxen der Formel 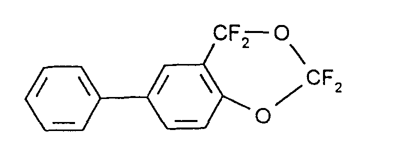 zu und rührt 12 Stunden bei Raumtemperatur. Das dunkelbraune Reaktionsgemisch wird auf 450 ml Eiswasser gegossen, der beige Niederschlag abfiltiert, mit Wasser gewaschen und getrocknet. Man erhält 22,3 g (90 % d.Th.) hellbraune Kristalle vom Fp. 193°C (Zersetzung).

### Beispiel (XIV-2)

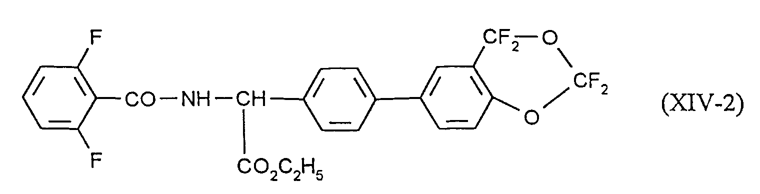

21,1 g (0,0425 Mol) Produkt aus Beispiel (XIV-1) werden in 170 ml trockenem Ethanol vorgelegt. Zu der klaren Lösung tropft man in 10 Minuten bei Raumtemperatur beginnend (exotherme Reaktion) 7 g (0,0585 Mol) Thionylchlorid. Man erhitzt 4 Stunden zum Sieden, kühlt ab und engt ein. Man erhält 26,7 g eines dunkelbraunen Öls, das über Kieselgel (Laufmittel Methylenchlorid) gereinigt wird.
Ausbeute: 7,7 g (34,1 % d.Th.) gelbe Kristalle v. Fp. 110-113°C.

### Beispiel (III-1)

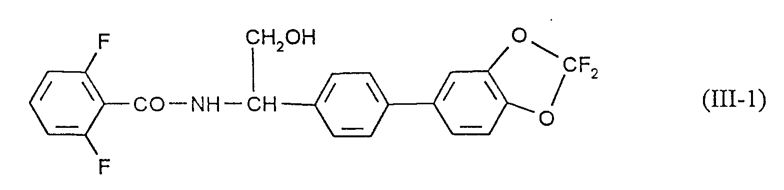

6,5 g (0,028 Mol) α-Hydroxy-N-(2,6-difluorbenzoyl)-glycin der Formel 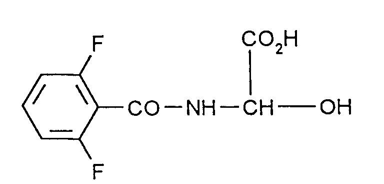 werden in 50 ml Methansulfonsäure gelöst. Bei 10°C werden 6,55 g (0,028 Mol) 5-Phenyl-2,2-difluor-benzodioxol der Formel 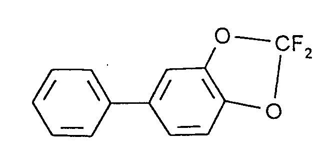 hinzugegeben. Man rührt 10 Stunden bei Raumtemperatur. Die braune Suspension wird auf 250 ml Eiswasser gegossen. Der beige Niederschlag wird abfiltriert, mit Wasser gewaschen und getrocknet.

Die Kristalle werden in 100 ml Ethanol suspendiert und 4,5 g Thionylchlorid zugetropft. Man erhitzt zum Sieden und hält 4 h bei dieser Temperatur. Man verdünnt mit 100 ml Wasser und gibt diese Lösung zu 5,32 g (0,14 Mol) Natriumboranat in 50 ml wäßrigem Ethanol. Nach beendeter Zugabe erhitzt man 4 Stunden zum Sieden, kühlt auf 5°C ab und filtriert ab, versetzt mit 2 n Salzsäure und extrahiert dreimal mit Methylenchlorid, trocknet die organische Phase und engt ein.
Ausbeute: 5,3 g (43,6 % d.th.) farblose Kristalle vom Fp. 196-201°C.

### Beispiel (III-2)

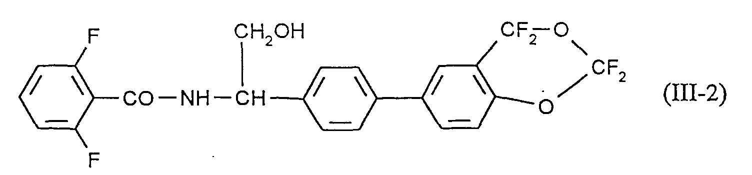

2,5 g (0,0665 Mol) Natriumboranat werden in 65 ml 50 %igem wäßrigem Ethanol vorgelegt, dann 7 g (0,0133 Mol) Produkt aus Beispiel (XIV-2) portionsweise zugegeben und 4 Stunden zum Sieden erhitzt. Man engt etwas ein, versetzt mit 100 ml 2n Salzsäure und saugt ab: 6,7 g (72,8 % d.Th.) weiße Kristalle.

**Beispiel (II-1)**

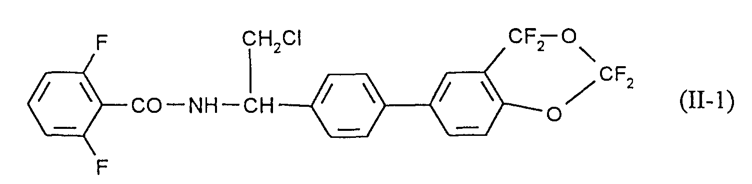

6,5 g (0,0135 Mol) Produkt aus Beispiel (III-2) wird in 70 ml trockenem Toluol suspendiert. Man tropft dann bei Raumtemperatur beginnend 6,4 g (0,054 Mol) Thionylchlorid zu und rührt 3 Stunden bei 70°C nach. Nach dem Einengen bleiben 6,6 g rohes Produkt der Formel (II-1) zurück, das roh in Beispiel (I-2) eingesetzt wird.

In den folgenden Anwendungsbeispiele wurde die aus der EP-A 0 432 661 bekannte Verbindung der Formel 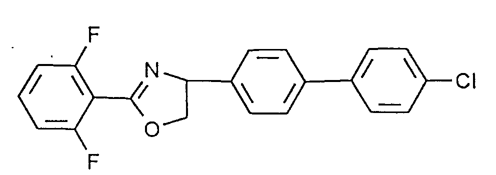 als Vergleichssubstanz eingesetzt.

### Beispiel A

| Plutella-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test zeigte z.B. die Verbindung gemäß Herstellungsbeispiel (I-2) überlegene Wirksamkeit gegenüber der Vergleichssubstanz.

### Beispiel B

| Spodoptera-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test zeigte z.B. die Verbindung gemäß Herstellungsbeispiel (I-2) überlegene Wirksamkeit gegenüber der Vergleichssubstanz.

## Patentansprüche

1. Verbindungen der Formel (I) 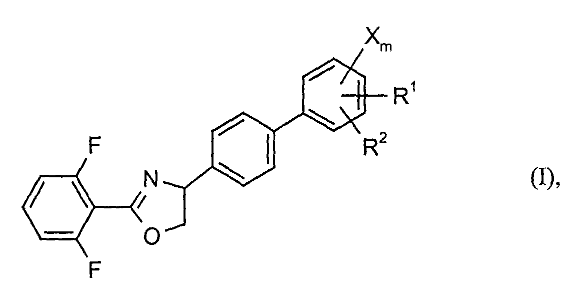 in welcher
R¹ für C₁-C₆-Halogenalkylthio steht und
R² für Wasserstoff steht oder
R¹ und R² gemeinsam mit den Kohlenstoffatomen, an die sie gebunden sind einen halogensubstituierten 5- oder 6-gliedrigen heterocyclischen Ring bilden,
X für Wasserstoff, Halogen, C₁-C₆-Alkyl oder C₁-C₆-Alkoxy steht und
m für 0, 1 oder 2 steht,
ausgenommen die Verbindung der Formel

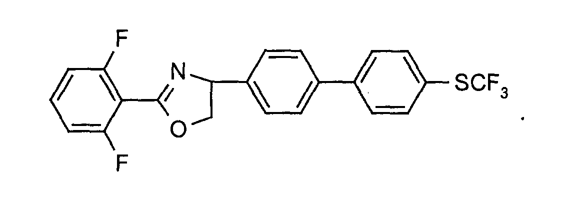

2. Verbindungen der Formel (I) gemäß Anspruch 1, in welcher
R¹ für C₁-C₄-Halogenalkylthio und
R² für Wasserstoff steht oder
R¹ und R² gemeinsam mit den einander direkt benachbarten Kohlenstoffatomen, an die sie gebunden sind, einen sauerstoffhaltigen 5- oder 6-gliedrigen Ring bilden, der einfach oder mehrfach durch Fluor und/oder Chlor substituiert ist,
X für Fluor oder Chlor steht und
m für 0 oder 1 steht,
ausgenommen die Verbindung der Formel

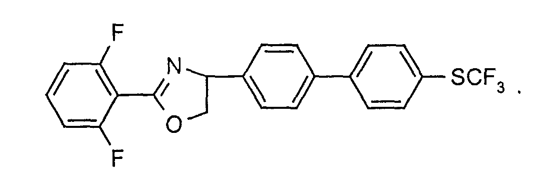

3. Verbindungen der Formel (I) gemäß Anspruch 1, in welcher
R¹ für SCHF₂, SCF₂CHFCl, SCF₂CF₂H, SCF₂Cl, SCF₂Br, SCF₂CH₂F, SCF₂CF₃, SCF₂CHCl₂, SCH₂CF₂CHF₂, SCH₂CF₂CF₃ oder SCF ₂CHFCF₃, und
R² für Wasserstoff steht oder,
R¹ und R² an direkt benachbarte Kohlenstoffatome gebunden sind und gemeinsam für
-OCF₂O-, -OCF₂CF₂O-, -OCF₂CFClO-,
-OCF₂OCF₂ oder -OCF₂CF₂- stehen,
X für Fluor oder Chlor steht und
m für 0 oder 1 steht.

4. Verfahren zur Herstellung der Verbindungen der Formel (I) gemäß Anspruch 1, **dadurch gekennzeichnet, daß** man Verbindungen der Formel (II) 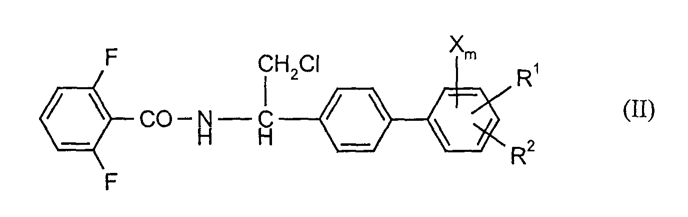 in welcher
R¹, R², X und m die in Anspruch 1 angegebene Bedeutung haben,
in Gegenwart einer Base, gegebenenfalls in Gegenwart eines Katalysators und gegebenenfalls in Gegenwart eines Verdünnungsmittels cyclisiert.

5. Verbindungen der Formel (X) 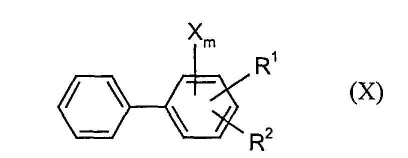 in welcher
R¹, R², X und m die in Anspruch 1 angegebene Bedeutung haben.

6. Verbindungen der Formel (IX) 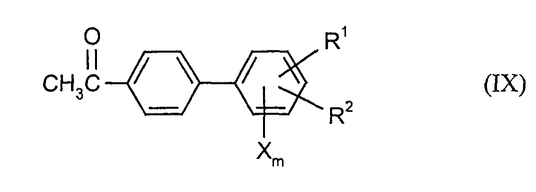 in welcher
R¹, R², X und m die in Anspruch 1 angegebene Bedeutung haben.

7. Verbindungen der Formel (VIII) 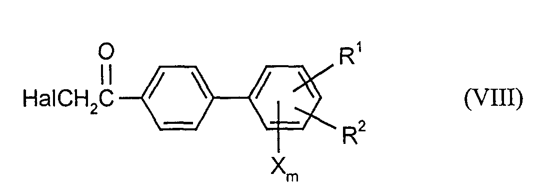 in welcher
R¹, R², X und m die oben angegebene Bedeutung haben und
Hal für Chlor oder Brom steht.

8. Verbindungen der Formel (VI) 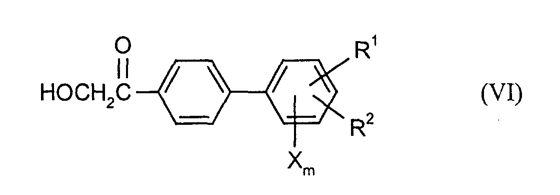 in welcher
R¹, R², X und m die in Anspruch 1 angegebene Bedeutung haben.

9. Verbindungen der Formel (V) 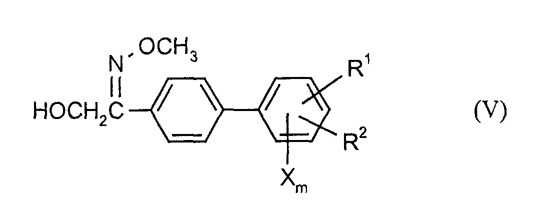 in welcher
R¹, R², X und m die in Anspruch 1 angegebene Bedeutung haben.

10. Verbindungen der Formel (IV) 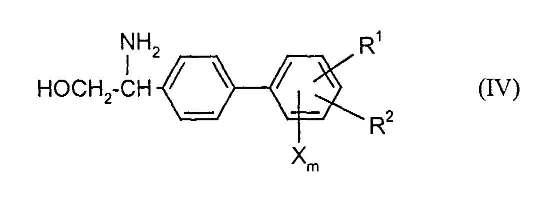 in welcher
R¹, R², X und m die in Anspruch 1 angegebene Bedeutung haben.

11. Verbindungen der Formel (III) 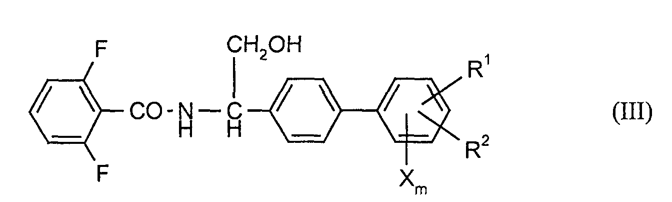 in welcher
R¹, R², X und m die in Anspruch 1 angegebene Bedeutung haben.

12. Verbindungen der Formel (XIV) 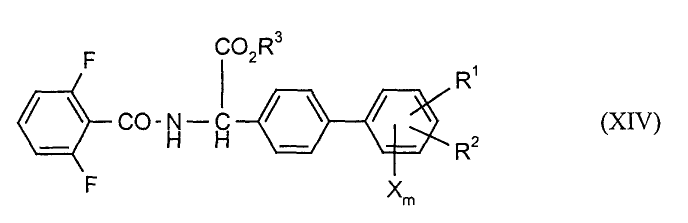 in welcher
R¹, R², X und m die in Anspruch 1 angegebene Bedeutung haben und
R³ für Alkyl steht.

13. Verbindungen der Formel (II) 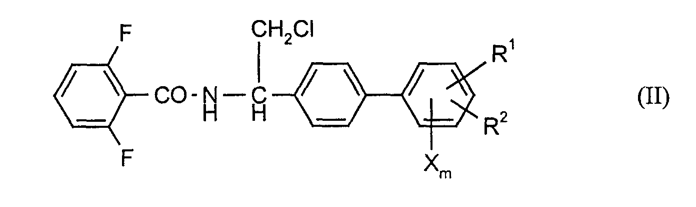 in welcher
R¹, R², X und m die in Anspruch 1 angegebene Bedeutung haben.

14. Schädlingsbekämpfungsmittel, **gekennzeichnet durch** einen Gehalt an mindestens einer Verbindung der Formel (I) gemäß Anspruch 1.

15. Verwendung von Verbindungen der Formel (I) gemäß Anspruch 1 zur Bekämpfung von Schädlingen.

16. Verfahren zur Bekämpfung von Schädlingen, **dadurch gekennzeichnet, daß** man Verbindungen der Formel (I) gemäß Anspruch 1 auf Schädlinge und/oder ihren Lebensraum einwirken läßt.

17. Verfahren zur Herstellung von Schädlingsbekämpfungsmitteln, **dadurch gekennzeichnet, daß** man Verbindungen der Formel (I) gemäß Anspruch 1 mit Streckmitteln und/oder oberflächenaktiven Mitteln vermischt.

18. Verwendung von Verbindungen der Formel (I) gemäß Anspruch 1 zur Herstellung von Schädlingsbekämpfungsmitteln.

19. Verbindung der Formel

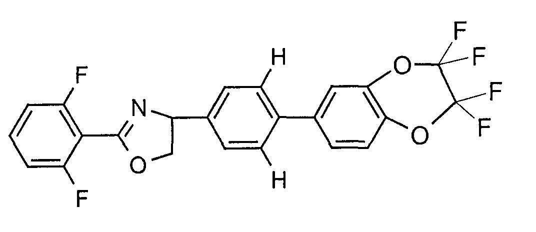

## Claims

1. A compound of the formula (I) 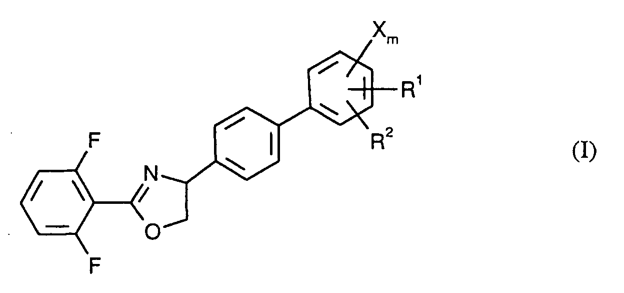 in which
R¹ represents C₁-C₆-halogenoalkylthio and
R² represents hydrogen, or
R¹ and R² together with the carbon atoms to which they are bonded form a halogen-substituted 5- or 6-membered heterocyclic ring,
X represents hydrogen, halogen, C₁-C₆-alkyl or C₁-C₆-alkoxy, and
m represents 0, 1 or 2,
with the exception of the compound of the formula

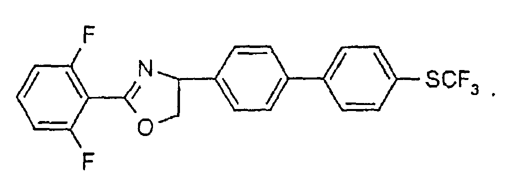

2. A compound of the formula (I) as claimed in claim 1, in which
R¹ represents C₁-C₄-halogenoalkylthio and
R² represents hydrogen, or
R¹ and R² together with the mutually directly adjacent carbon atoms to which they are bonded form an oxygen-containing 5- or 6-membered ring which is monosubstituted or polysubstituted by fluorine and/or chlorine,
X represents fluorine or chlorine and
m represents 0 or 1,
with the exception of the compound of the formula

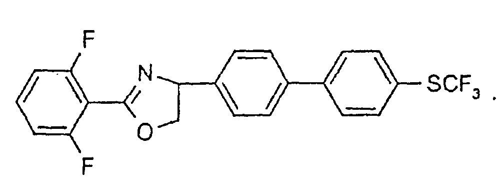

3. A compound of the formula (I) as claimed in claim 1, in which
R¹ represents SCHF₂, SCF₂CHFCl, SCF₂CF₂H, SCF₂Cl, SCF₂Br, SCF₂CH₂F, SCF₂CF₃, SCF₂CHCl₂, SCH₂CF₂CHF₂, SCH₂CF₂CF₃ or SCF₂CHFCF₃, and
R² represents hydrogen, or
R¹ and R² are bonded to directly adjacent carbon atoms and together represent -OCF₂O-, -OCF₂CF₂O-, -OCF₂CFClO-, -OCF₂OCF₂ or -OCF₂CF₂-,
X represents fluorine or chlorine and
m represents 0 or 1.

4. A process for the preparation of the compound of the formula (I) as claimed in claim 1, **characterized in that** it comprises cyclizing compounds of the formula (II) 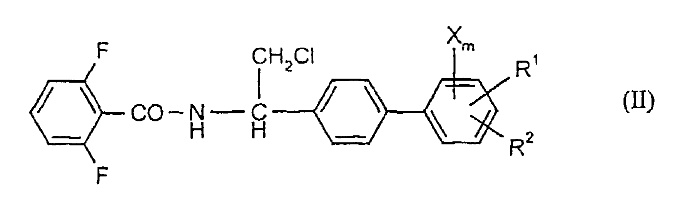 in which
R¹, R², X and m have the meanings given in claim 1,
in the presence of a base, if appropriate in the presence of a catalyst and, if appropriate, in the presence of a diluent.

5. A compound of the formula (X) 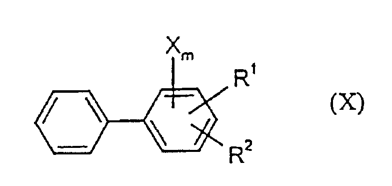 in which
R¹, R², X and m have the meanings given in claim 1.

6. A compound of the formula (IX) 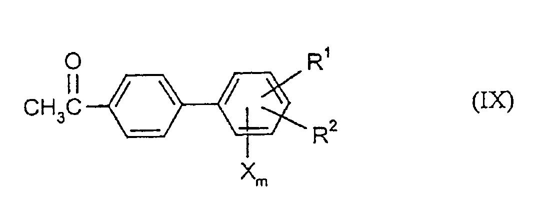 in which
R¹, R², X and m have the meanings given in claim 1.

7. A compound of the formula (VIII) 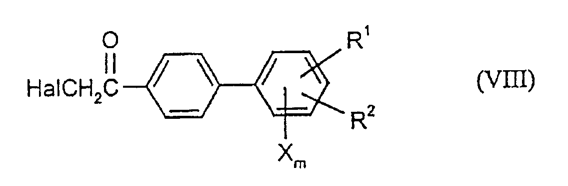 in which
R¹, R², X and m have the abovementioned meanings and
Hal represents chlorine or bromine.

8. A compound of the formula (VI) 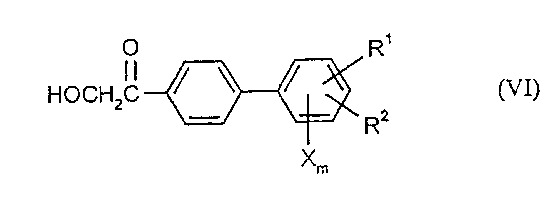 in which
R¹, R², X and m have the meanings given in claim 1.

9. A compound of the formula (V) 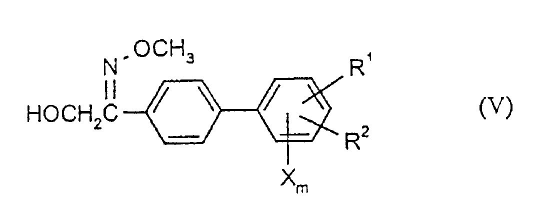 in which
R¹, R², X and m have the meanings given in claim 1.

10. A compound of the formula (IV) 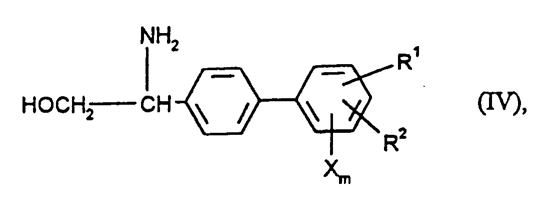 in which
R¹, R², X and m have the meanings given in claim 1.

11. A compound of the formula (III) 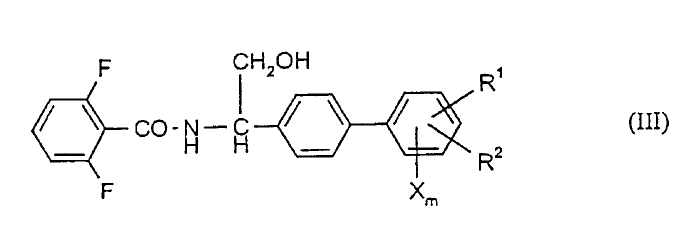 in which
R¹, R², X and m have the meanings given in claim 1.

12. A compound of the formula (XIV) 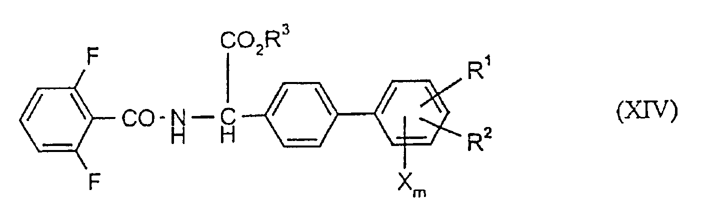 in which
R¹, R², X and m have the meanings given in claim 1 and
R³ represents alkyl.

13. A compound of the formula (II) 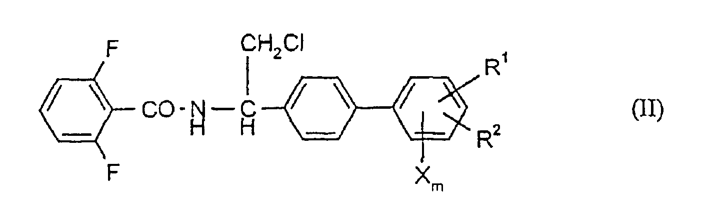 in which
R¹, R², X and m have the meanings given in claim 1.

14. A pesticide **characterized by** containing at least one compound of the formula (I) as claimed in claim 1.

15. The use of a compound of the formula (I) as claimed in claim 1 for combating pests.

16. A method of combating pests, **characterized in that** a compound of the formula (I) as claimed in claim 1 is allowed to act on pests and/or their environment.

17. A process for the preparation of pesticides, **characterized in that** a compound of the formula (I) as claimed in claim 1 is mixed with extenders and/or surface-active agents.

18. The use of a compound of the formula (I) as claimed in claim 1 for the preparation of pesticides.

19. A compound of the formula (IIa)

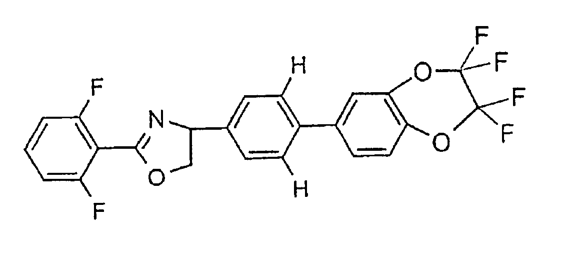

## Revendications

1. Composés répondant à la formule (I) 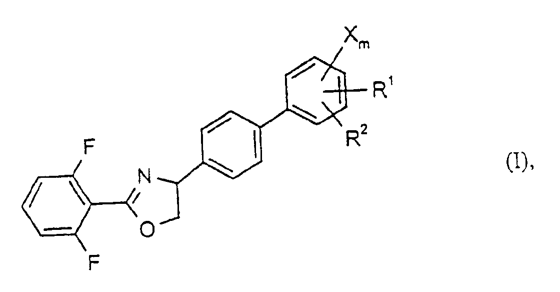 dans laquelle
R¹ représente un groupe halogénalkyl(en C₁-C₆)thio et
R² représente un atome d'hydrogène, ou bien
R¹ et R² représentent, ensemble avec les atomes de carbone auxquels ils sont liés, un noyau hétérocyclique penta- ou hexagonal portant un ou plusieurs substituants halogéno,
X représente un atome d'hydrogène, un atome d'halogène, un groupe alkyle en C₁-C₆ ou un groupe alcoxy en C₁-C₆ et
m représente 0, 1 ou 2,
à l'exception du composé répondant à la formule

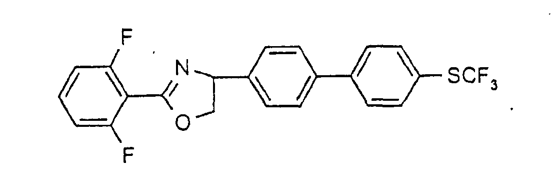

2. Composés répondant à la formule (I) selon la revendication 1, dans lesquels
R¹ représente un groupe halogénalkyl(en C₁-C₄)thio et
R² représente un atome d'hydrogène, ou bien
R¹ et R² représentent, ensemble avec les atomes de carbone directement voisins l'un de l'autre auxquels ils sont liés, un noyau hétérocyclique penta- ou hexagonal oxygéné qui porte un ou plusieurs substituants fluoro et/ou chloro,
X représente un atome de fluor ou un atome de chlore, et
m représente 0 ou 1,
à l'exception du composé répondant à la formule

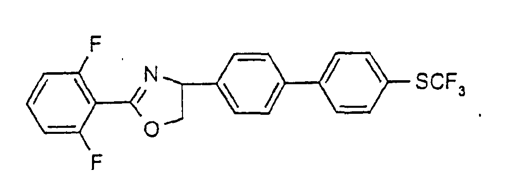

3. Composés répondant à la formule (I) selon la revendication 1, dans lesquels
R¹ représente un groupe SCHF₂, un groupe SCF₂CHFCl, un groupe SCF₂CF₂H, un groupe SCF₂Cl, un groupe SCF₂Br, un groupe SCF₂CH₂F, un groupe SCF₂CF₃, un groupe SCF₂CHCl₂, un groupe SCH₂CF₂CHF₂, un groupe SCH₂CF₂CF₃, un groupe SCF₂CHFCF₃, et
R² représente un atome d'hydrogène, ou bien
R¹ et R² sont liés à des atomes de carbone directement voisins et représentent ensemble un groupe -OCF₂O-, un groupe -OCF₂CF₂O-, un groupe -OCF₂CFClO-, un groupe -OCF₂OCF₂- ou un groupe -OCF₂CF₂-,
X représente un atome de fluor ou un atome de chlore, et
m représente 0 ou 1.

4. Procédé pour la préparation des composés répondant à la formule (I) selon la revendication 1, **caractérisé en ce qu'**on soumet à une cyclisation, des composés répondant à la formule (II) 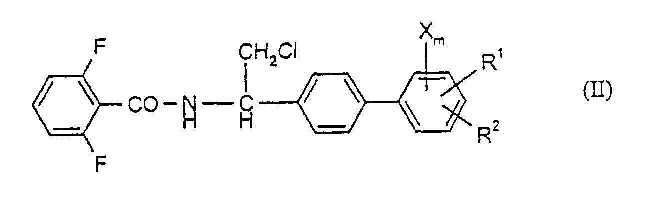 dans laquelle
R¹, R², X et m ont la signification indiquée à la revendication 1,
en présence d'une base, le cas échéant, en présence d'un catalyseur et, le cas échéant, en présence d'un diluant.

5. Composés répondant à la formule (X) 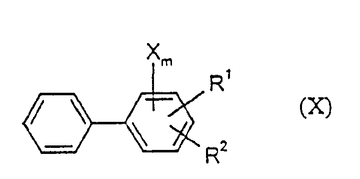 dans laquelle
R¹, R², X et m ont la signification indiquée à la revendication 1.

6. Composés répondant à la formule (IX) 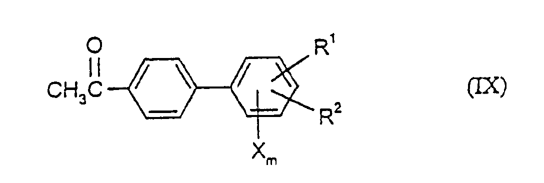 dans laquelle
R¹, R², X et m ont la signification indiquée à la revendication 1.

7. Composés répondant à la formule (VIII) 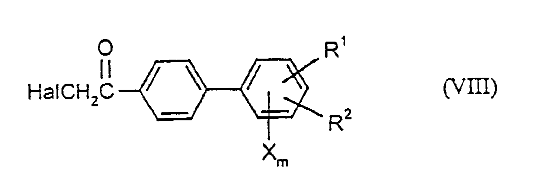 dans laquelle
R¹, R², X et m ont la signification indiquée ci-dessus et
Hal représente un atome de chlore ou un atome de brome.

8. Composés répondant à la formule (VI) 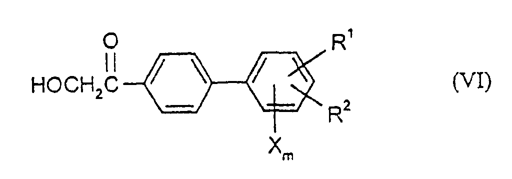 dans laquelle
R¹, R², X et m ont la signification indiquée à la revendication 1.

9. Composés répondant à la formule (V) 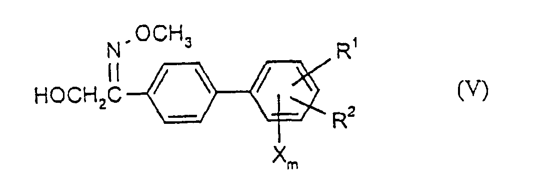 dans laquelle
R¹, R², X et m ont la signification indiquée à la revendication 1.

10. Composés répondant à la formule (IV) 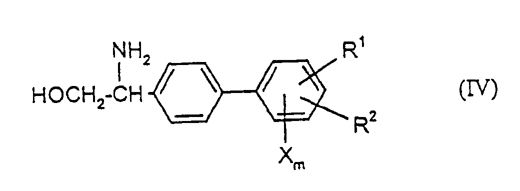 dans laquelle
R¹, R², X et m ont la signification indiquée à la revendication 1.

11. Composés répondant à la formule (III) 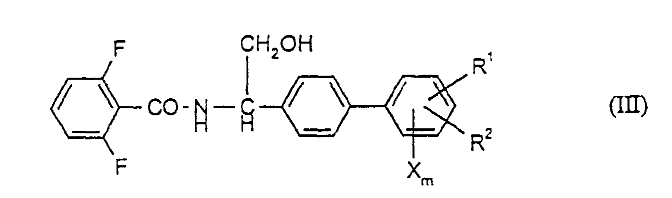 dans laquelle
R¹, R², X et m ont la signification indiquée à la revendication 1.

12. Composés répondant à la formule (XIV) 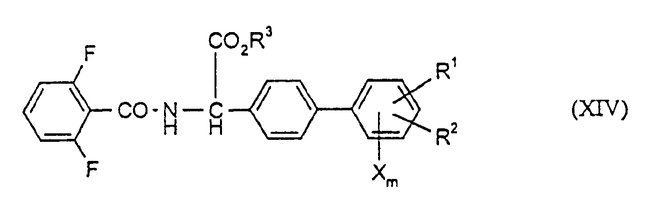 dans laquelle
R¹, R², X et m ont la signification indiquée à la revendication 1 et
R³ représente un groupe alkyle.

13. Composés répondant à la formule (II) 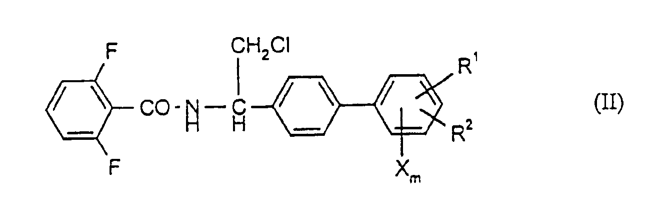 dans laquelle
R¹, R², X et m ont la signification indiquée à la revendication 1.

14. Agent de lutte contre les parasites, **caractérisé par** une teneur en au moins un composé répondant à la formule (I) selon la revendication 1.

15. Utilisation de composés répondant à la formule (I) selon la revendication 1 pour lutter contre les parasites.

16. Procédé pour lutter contre les parasites, **caractérisé en ce qu'**on laisse agir des composés répondant la formule (I) selon la revendication 1 sur des parasites et/ou sur leur biotope.

17. Procédé pour la préparation d'agents de lutte contre les parasites, **caractérisé en ce qu'**on mélange des composés répondant à la formule (I) selon la revendication 1 avec des diluants et/ou des agents tensioactifs.

18. Utilisation de composés répondant à la formule (I) selon la revendication 1 pour la préparation d'agents de lutte contre les parasites.

19. Composé répondant à la formule
